(19) Europäisches Patentamt

European Patent Office

Office européen des brevets

(11) Veröffentlichungsnummer: **0 403 950 B1**

(12) **EUROPÄISCHE PATENTSCHRIFT**

(45) Veröffentlichungstag der Patentschrift: **15.02.95**

(21) Anmeldenummer: **90111186.4**

(22) Anmeldetag: **13.06.90**

(51) Int. Cl.6: **C07C 69/15**, C07C 67/055, B01J 29/70

(54) **Verfahren zur Herstellung von Vinylacetat.**

(30) Priorität: **15.06.89 DE 3919524**

(43) Veröffentlichungstag der Anmeldung:
**27.12.90 Patentblatt 90/52**

(45) Bekanntmachung des Hinweises auf die
Patenterteilung:
**15.02.95 Patentblatt 95/07**

(84) Benannte Vertragsstaaten:
**BE DE ES FR GB IT LU NL**

(56) Entgegenhaltungen:
EP-A- 82 222          EP-A- 0 330 853
DE-A- 1 793 447      DE-A- 2 528 363
GB-A- 1 500 167      US-A- 3 939 199
US-A- 4 048 096

**WORLD PATENT INDEX, accession no.
77-26067y (15), classes A41 E17, Derwent
Publications, Ltd., London, GB; & JP-A-52 027
710**

**Patent Abstracts of Japan,11 (85),
(C410)[2532], JP-A- 61 238 759**

**Patent Abstract of Japan, 9 (228),
(C303)[1951], JP-A- 60 87 857**

(73) Patentinhaber: **HOECHST AKTIENGESELL-
SCHAFT**

**D-65926 Frankfurt (DE)**

(72) Erfinder: **Wunder, Friedrich, Dr.
Jahnstrasse 46
D-6093 Flörsheim am Main (DE)**
Erfinder: **Wirtz, Peter, Dr.
Wiesbadener Strasse 135
D-6240 Königstein/Taunus (DE)**
Erfinder: **Eichler, Klaus, Dr.
Im Traminer 10
D-6236 Eschborn (DE)**
Erfinder: **Roscher, Günter, Dr.
Hölderlinstrasse 64
D-6233 Kelkheim (Taunus) (DE)**

**Beschreibung**

Es ist bekannt, daß man Ethylen in der Gasphase mit Essigsäure und Sauerstoff oder sauerstoffhaltigen Gasen an Festbettkatalysatoren Zu Vinylacetat umsetzen kann. Geeignete Katalystoren enthalten einen Edelmetallanteil und einen Aktivatoranteil. Der Edelmetallanteil besteht vorzugsweise aus Palladium und/oder dessen Verbindungen; zusätzlich können noch Gold und/oder dessen Verbindungen anwesend sein (US-PS 3 939 199, DE-OS 2 100 778, US-PS 4 668 819). Der Aktivatoranteil besteht dabei aus Verbindungen von Elementen der 1. Hauptgruppe und/oder der 2. Hauptgruppe und/oder Cadmium. Bevorzugt ist Kalium als Element der 1. Hauptgruppe. Diese aktiven Komponenten werden in feiner Verteilung auf Träger aufgebracht, wobei als Trägermaterial im allgemeinen Kieselsäure oder Aluminiumoxid verwendet wird.

Die spezifische Oberfläche der Träger liegt im allgemeinen bei 40 - 350 $m^2$/g. Nach US-PS 3 939 199 soll das Gesamtporenvolumen bei 0,4 - 1,2 ml/g liegen, und davon sollen weniger als 10 % von "Mikroporen" mit einem Porendurchmesser unter 30 Angström gebildet werden. Geeignete Träger mit diesen Eigenschaften sind z.B. aerogenes $SiO_2$ oder aerogenes $SiO_2$-$Al_2O_3$-Gemisch. Die Trägerteilchen bei der Vinylacetatherstellung haben im allgemeinen die Form von Kugeln. Aber auch Tabletten und Zylinder wurden bereits eingesetzt.

Aus DE-A-2 528 363 ist ein metallisches Gold und Palladium enthaltender Trägerkatalysator mit einem hohen Anteil an Poren im Bereich von 100 Angström bekannt, bei dem mehr als 90 Gew.% jedes der beiden Edelmetalle im äußeren Teil der Trägerteilchen vorliegen, der bis zu einer Tiefe von 30% der Trägerteilchen reicht.

Ein Gegenstand der Erfindung ist ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, sowie gegebenenfalls zusätzlich Gold und/oder Goldverbindungen, sowie als Aktivatoren Alkaliverbindungen und gegebenenfalls zusätzlich Cadmiumverbindungen auf einem Träger enthält, der aus $SiO_2$ oder $SiO_2$-$Al_2O_3$-Gemisch mit einer Oberfläche von 50 - 250 $m^2$/g und einem Porenvolumen von 0,4 - 1,2 ml/g besteht und eine Korngröße von 4 bis 9 mm hat, dadurch gekennzeichnet, daß 5 bis 20 % des Porenvolumens des Trägers von Poren mit Radien von 200 bis 3000 Angström und 50 bis 90 % des Porenvolumens von Poren mit Radien von 70 bis 100 Angström gebildet wird, wobei Katalysatoren ausgeschlossen sind, die Palladium und Gold als Metalle enthalten und mehr als 90 Gew.% jedes dieser beiden Metalle im äußeren Teil der Trägerteilchen vorliegen, der bis zu einer Tiefe von 30% der Trägerteilchen reicht.

Weitere Gegenstände der Erfindung sind ein entsprechender Katalysator sowie ein Verfahren zu dessen Herstellung.

Vorzugsweise werden 8 bis 15 % des Porenvolumens des Trägers von Poren mit Radien von 200 bis 3000 Angström gebildet und 55 - 75 % des Porenvolumens von Poren mit Radien von 70 bis 100 Angström.

Derartige Träger werden wie folgt erhalten: Zunächst werden glasige Mikrokugeln hergestellt, beispielsweise durch Flammenhydrolyse von Siliciumtetrachlorid oder einem Siliciumtetrachlorid-Aluminiumtrichlorid-Gemisch in einer Knallgasflamme (US-PS 3 939 199). Die Mikrokugeln können auch durch Schmelzen von feinstem $SiO_2$-Staub in einer ausreichend heißen Flamme und anschließende rasche Abkühlung hergestellt werden. Die auf eine der beiden Arten hergestellten Mikrokugeln haben eine Oberfläche von 100 - 300 $m^2$/g. Besonders geeignet sind Mikrokugeln mit einer Oberfläche von 150 - 250 $m^2$/g, die aus mindestens 95 Gew.-% $SiO_2$ und höchstens 5 Gew.-% $Al_2O_3$, insbesondere aus mindestens 99 Gew.-% $SiO_2$ und höchstens 1 Gew.-% $Al_2O_3$ bestehen. Mikrokugeln mit der genannten Oberfläche sind im Handel erhältlich, z.B. unter dem Namen [R]Aerosil oder [R]Cabosil oder als "hochdisperse Kieselsäure".

Aus den Mikrokugeln werden dann unter Verwendung von organischen Füllstoffen (wie Zucker, Harnstoff, höhere Fettsäuren, längerkettige Paraffine, mikrokristalline Zellulose) und Gleitmitteln (wie Kaolin, Graphit, Metallseifen) Formkörper gepreßt, z.B. durch Tablettieren (nach einer Vorverdichtung) oder Extrudieren. Durch Glühen dieser Formkörper in $O_2$-haltigen Gasen werden diese Hilfsmittel anschließend wieder entfernt. Die Oberfläche des fertigen Trägers, sein Porenvolumen und der Anteil des Porenvolumens, den Poren eines bestimmten Radius bilden ("Porenradienverteilung"), wird determiniert von der Art der Verformung (Tabletten, Strangpreßlinge usw.), der Temperatur und Dauer des Glühens, den relativen Mengen von Füllstoffen, Gleitmitteln und Mikrokugeln, sowie der Oberfläche der Mikrokugeln. Welches die geeignetsten Werte für diese determinierenden Parameter sind, läßt sich durch einfache Vorversuche ermitteln.

Man kann auch auf Gleit- und Füllmittel verzichten und stattdessen ein Kieselsol mit den Mikrokugeln versetzen, dann trocknen und glühen. Diese Methode eignet sich besonders für Strangpreßlinge. Bei dieser Methode werden Oberfläche, Porenvolumen und "Porenradienverteilung" (siehe oben) determiniert durch die folgenden Parameter: Art des verwendeten Kieselsols (Größe der Primärteilchen, meßbar am Tyndall-Effekt), Art der verwendeten Mikrokugeln, Trocknungsgeschwindigkeit und - temperatur, sowie Glühdauer und -temperatur. Wieder lassen sich die geeignetsten Werte für diese Parameter durch einfache Vorversuche ermitteln.

Der nach einer der beiden Methoden erhaltene fertige Träger hat dann eine Oberfläche von 50 bis 250 $m^2/g$ und ein Porenvolumen von 0,4 bis 1,2 ml/g, sowie eine Korngröße von 4 bis 9 mm (einstellbar durch Tablettieren oder Extrudieren).

Durch Verwendung von Trägern mit der genannten speziellen Porenradienverteilung gelingt es, die Raum-Zeit-Ausbeute der Katalysatoren gegenüber konventionellen Trägern bei sonst gleichen Bedingungen (gleicher Gehalt an aktiven Substanzen auf dem Träger und gleichen Reaktionsbedingungen) wesentlich zu steigern und gleichzeitig die stärkste Nebenreaktion, die Verbrennung des Ethylens zu $CO_2$, um mehr als 30 % zu senken. Ebenso wird die als weitere Nebenreaktion ablaufende Ethylacetatbildung deutlich verringert. Die Vorteile des erfindungsgemäßen Verfahrens liegen darin, daß durch diese Steigerung der Selektivität von ca. 92 % auf ca. 95 % wesentliche Einsparungen erzielt werden, und daß außerdem durch die Leistungssteigerung bei deutlich erhöhter Selektivität in Neuanlagen Katalysatormenge und Reaktorvolumen verringert werden können, was zu beträchtlichen Verringerungen der Anlagekosten führt, oder daß bei bereits bestehenden Anlagen die Kapazität ohne Umbau wesentlich erhöht werden kann und man so die Investitionskosten für die Anlagenerweiterung einspart.

Bei der Oberfläche der genannten Träger handelt es sich stets um die sogenannte BET-Oberfläche, gemessen nach der Methode von Brunauer, Emmett und Teller. Sie gibt die Gesamtoberfläche von 1 g Trägermaterial an, d.h. die Summe aus der äußeren Oberfläche des Trägers und der inneren Oberfläche sämtlicher offenen Poren. Das gesamte Porenvolumen und der Anteil davon, den Poren bestimmter Größe (z.B. diejenigen mit einem Durchmesser von 70 bis 100 Angström) beitragen, läßt sich mit Hilfe der Quecksilber-Porosimetrie messen. Geeignete Meßapparaturen werden z.B. von den Firmen Carlo Erba oder Micromeritics hergestellt.

Die katalytisch aktiven Substanzen werden in üblicher Weise auf den Träger aufgebracht, beispielsweise durch Tränken des Trägers mit einer Lösung der aktiven Substanzen, anschließende Trocknung und gegebenenfalls Reduktion. Jedoch kann man die aktiven Substanzen auch beispielsweise durch Ausfällung auf dem Träger, durch Aufsprühen, Aufdampfen, Tauchen aufbringen.

Als Lösungsmittel für die katalytisch aktiven Substanzen sind vor allem unsubstituierte Carbonsäuren mit 2 bis 10 Kohlenstoffatomen im Molekül, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird vorzugsweise Essigsäure als Lösungsmittel eingesetzt. Die zusätzliche Verwendung eines inerten Lösungsmittels ist dann zweckmäßig, wenn die Substanzen in der Carbonsäure nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind. Genannt seien neben Wasser beispielsweise Ketone wie Aceton und Acetylaceton, ferner Ether wie Tetrahydrofuran oder Dioxan, aber auch Kohlenwasserstoffe wie Benzol.

Der Katalysator enthält als Edelmetallkomponente Palladium und/oder dessen Verbindungen und als Aktivatorkomponente Alkaliverbindungen. Als zusätzliche Edelmetallkomponente kann er Gold und/oder dessen Verbindungen enthalten, und als zusätzliche Aktivatorkomponente kann er Cadmiumverbindungen enthalten.

Als Verbindungen des Palladiums kommen alle Salze und Komplexe in Betracht, die löslich (sowie gegebenenfalls reduzierbar) sind und im fertigen Katalysator keine desaktivierenden Stoffe wie Halogen oder Schwefel hinterlassen. Besonders geeignet sind die Carboxylate, vorzugsweise die Salze der aliphatischen Monocarbonsäuren mit 1 bis 5 Kohlenstoffatomen, etwa das Acetat, das Propionat oder das Butyrat. Weiter sind beispielsweise geeignet das Nitrat, Nitrit, Oxidhydrat, Oxalat, Acetylacetonat oder das Acetoacetat. Aber auch Verbindungen wie das Sulfat und die Halogenide können verwendet werden, wenn man dafür Sorge trägt, daß der Sulfatrest, z.B. durch Fällen mit Bariumacetat, oder das Halogen, z.B. durch Fällen mit Silbernitrat, vor der Tränkung entfernt wird, so daß das Sulfat- oder Halogenanion nicht auf den Träger gerät. Wegen seiner Löslichkeit und seiner Zugänglichkeit ist Palladiumacetat die besonders bevorzugte Palladiumverbindung.

Im allgemeinen liegt der Gehalt an Palladium im Katalysator bei 1,0 bis 3 Gew.-%, vorzugsweise bei 1,5 bis 2,5 Gew.-%, insbesondere bei 2 bis 2,5 Gew.-%, wobei der Metallanteil auf die Gesamtmasse des Trägerkatalysators bezogen wird.

Neben Palladium und/oder dessen Verbindungen können noch zusätzlich Gold und/oder dessen Verbindungen anwesend sein. Eine besonders geeignete Goldverbindung ist Bariumacetoaurat. Im allgemeinen wird Gold bzw. eine seiner Verbindungen, falls es eingesetzt wird, in einem Anteil von 0,2 bis 0,7 Gew.-% zugegeben, wobei der Metallanteil auf die Gesamtmasse des Trägerkatalysators bezogen wird.

Als Aktivatoren enthält der Katalysator Alkaliverbindungen und gegebenenfalls zusätzlich Cadmiumverbindungen. Geeignet sind beispielsweise Alkalicarboxylate wie etwa Kaliumacetat, Natriumacetat, Lithiumacetat, Natriumpropionat. Geeignet sind auch solche Alkaliverbindungen, die unter Reaktionsbedingungen in die Carboxylate übergehen, wie etwa Hydroxide, Oxide, Carbonate. Als Verbindungen des Cadmiums kommen solche in Frage, die kein Halogen oder Schwefel enthalten, beispielsweise Carboxylat (bevorzugt), Oxid, Hydroxid, Carbonat, Citrat, Tartrat, Nitrat, Acetylacetonat, Benzoylacetonat, Acetoacetat. Besonders geeignet ist Cadmiumacetat. Auch Gemische verschiedener Aktivatoren lassen sich einsetzen. Jeder einzelne Aktivator wird im allgemeinen in einem Anteil von 0,5 - 4 Gew.-% zugegeben, wobei der Metallanteil des Aktivators auf die Gesamtmasse des Trägerkatalysators bezogen wird.

Bevorzugt sind folgende Katalysatoren: Palladium/Alkalielement/Cadmium sowie Palladium/Gold/Alkalielement, wobei Palladium bzw. Gold als Metalle oder Verbindungen im fertigen Katalysator vorliegen können und wobei als Alkalielement Kalium bevorzugt ist (in Form eines Carboxylats). Das Verhältnis K:Pd bzw. K:(Pd + Au) ist dabei vorzugsweise 0,7:1 bis 2:1. Das Verhältnis Cd:Pd bzw. Cd:(Pd + Au) ist vorzugsweise 0,6:1 bis 2:1, insbesondere 0,6:1 bis 0,9:1. Dabei werden Pd, Au, Cd, K stets als Elemente gerechnet, d.h. z.B. nur die Metallanteile von Pd-Acetat, Cd-Acetat und K-Acetat auf dem Träger miteinander verglichen.

Besonders bevorzugt sind die Katalysatoren Palladiumacetat/Kaliumacetat/Cadmiumacetat, sowie Palladiumacetat/Bariumacetoaurat/Kaliumacetat.

Die Tränkung des Katalysatorträgers mit der Lösung der aktiven Komponenten wird vorzugsweise so vorgenommen, daß das Trägermaterial mit der Lösung überschichtet und die überschüssige Lösung dann abgegossen oder abfiltriert wird. Mit Rücksicht auf Lösungsverluste ist es vorteilhaft, nur die dem integralen Porenvolumen des Katalysatorträgers entsprechende Lösung einzusetzen und sorgfältig durchzumischen, damit die Teilchen des Trägermaterials gleichmäßig benetzt werden. Diese Durchmischung läßt sich z.B. durch Rühren erreichen. Es ist zweckmäßig, den Tränkungsvorgang und das Durchmischen gleichzeitig durchzuführen, beispielsweise in einer Drehtrommel oder einem Taumeltrockner, wobei sich die Trocknung sofort anschließen kann. Weiterhin ist es zweckmäßig, die Menge und die Zusammensetzung der zum Tränken des Katalysatorträgers verwendeten Lösung so zu bemessen, daß sie dem Porenvolumen des Trägermaterials entspricht und daß durch einmaliges Tränken die gewünschte Menge aktiver Stoffe aufgebracht wird.

Die Trocknung des mit der Lösung der aktiven Stoffe getränkten Katalysatorträgers wird vorzugsweise unter vermindertem Druck durchgeführt. Die Temperatur bei der Trocknung sollte unter 120°C, vorzugsweise unter 90°C liegen. Weiterhin empfiehlt es sich im allgemeinen, die Trocknung in einem Inertgasstrom, beispielsweise in einem Stickstoff- oder Kohlendioxidstrom vorzunehmen. Der Lösungsmittel-Restgehalt nach der Trocknung sollte vorzugsweise weniger als 8 Gew.-%, insbesondere weniger als 6 Gew.-% betragen.

Falls eine Reduktion der Palladiumverbindungen (und ggf. der Goldverbindungen) durchgeführt wird, was manchmal nützlich sein kann, so kann diese im Vakuum, bei Normaldruck oder bei erhöhtem Druck bis zu 10 bar, ausgeführt werden. Dabei empfiehlt es sich, das Reduktionsmittel umso stärker mit einem Inertgas zu verdünnen, je höher der Druck ist. Die Reduktionstemperatur liegt zwischen 40 und 260°C, vorzugsweise zwischen 70 und 200°C. Im allgemeinen ist es zweckmäßig, für die Reduktion ein Inertgas-Reduktionsmittel-Gemisch zu verwenden, das 0,01 bis 50 Vol.-%, vorzugsweise 0,5 bis 20 Vol.-% Reduktionsmittel enthält. Als Inertgas können beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen und andere Olefine in Frage. Die Menge des Reduktionsmittels richtet sich nach der Menge des Palladiums und ggf. des eingesetzten Goldes; das Reduktionsäquivalent soll mindestens das 1- bis 1,5-fache des Oxidationsäquivalents betragen, jedoch schaden größere Mengen Reduktionsmittel nicht. Beispielsweise soll auf 1 Mol Palladium mindestens 1 Mol Wasserstoff verwendet werden. Die Reduktion kann im Anschluß an die Trocknung in der gleichen Anlage vorgenommen werden.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht-umgesetzte Komponenten im Kreis geführt werden können. Zweckmäßig hält man die Sauerstoffkonzentration unter 10 Vol.-% (bezogen auf das essigsäurefreie Gasgemisch). Unter Umständen ist jedoch auch eine Verdünnung mit inerten Gasen wie Stickstoff oder Kohlendioxid vorteilhaft. Besonders $CO_2$ eignet sich zur Verdünnung bei Kreisprozessen, da es in geringen Mengen während der Reaktion gebildet wird.

Die folgenden Beispiele sollen die Erfindung erläutern.

**Vergleichsbeispiel 1**

(Kugelförmige Trägerteilchen aus konventionellem Kieselgel)

Es wurden 200 g eines Kieselsäureträgers eingesetzt, der aus getemperten (800°C) Kieselgel-Kugeln von 5 - 8 mm Durchmesser bestand. Der (handelsübliche) Träger aus diesen kugelförmigen Teilchen hatte eine BET-Oberfläche von 169 $m^2$/g und ein Porenvolumen von 0,48 ml/g, das zu 8 % von Poren mit 70 - 100 Angström Durchmesser und zu 29 % von Poren mit 200 - 3000 Angström Durchmesser gebildet wurde. Der Träger wurde mit einer (diesem Porenvolumen entsprechenden) Lösung von 11,5 g Pd-Acetat, 10,0 g Cd-Acetat und 10,8 g K-Acetat in 66 ml Eisessig getränkt und bei 60°C unter Stickstoff bei einem Druck von 200 mbar bis zu einem Lösungsmittel-Restgehalt von 2 Gew.-% getrocknet. Dies ergab eine Dotierung von 2,3 Gew.-% Pd, 1,8 Gew.-% Cd, 2,0 Gew.-%K (Cd:Pd = 0,78:1, K:Pd = 0,87:1).

Es wurden 50 ml des fertigen Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150°C das umzusetzende Gas über den Katalysator geleitet. Dieses Gas bestand am Reaktoreingang aus 27 Vol.-% Ethylen, 55 Vol.-% $N_2$, 12 Vol.-% Essigsäure und 6 Vol.-% $O_2$. Die Ergebnisse sind aus der Tabelle ersichtlich.

**Vergleichsbeispiel 2**

(Kugelförmige Trägerteilchen aus konventionellem $SiO_2$)

Es wurden 200 g eines Kieselsäureträgers eingesetzt, der aus gebranntem und dann mit HCl gewaschenem Bentonit ($SiO_2$-Gehalt nach dieser Wäsche 96 Gew.-%) zu Kugeln von 5 -6 mm Durchmesser gepreßt worden war. Der Träger aus diesen kugelförmigen Teilchen hatte eine BET-Oberfläche von 121 $m^2$/g und ein Porenvolumen von 0,66 ml/g, das zu 21 % von Poren mit 70 - 100 Angström Durchmesser und zu 42 % von Poren mit 200 - 3000 Angström Durchmesser gebildet wurde. Die Trägerteilchen wurden wie in Vergleichsbeispiel 1 getränkt (nur daß 114 ml statt 66 ml Eisessig verwendet wurden) und getrocknet, so daß die gleiche Dotierung vorlag. Anschließend wurde der Katalysator wie in Vergleichsbeispiel 1 getestet. Die Ergebnisse sind aus der Tabelle ersichtlich.

**Beispiel 1**

Es wurde zunächst aus $SiO_2$-Mikrokugeln mit einer Oberfläche von 200 $m^2$/g sowie einem Füllstoff und einem Gleitmittel ein Träger hergestellt. Der fertige Träger hatte ein Porenvolumen von 0,80 ml/g, das zu 62 % von Poren mit 70 - 100 Angström Durchmesser und zu 9 % von Poren mit 200 - 3000 Angström Durchmesser gebildet wurde. Die Trägerteilchen hatten die Form von Zylindern mit gewölbten Stirnflächen (6 mm Durchmesser und 6 mm Höhe; die Form ist ähnlich der Form der bekannten Arzneimittelkapseln). Die Oberfläche der Trägerteilchen betrug 185 $m^2$/g.

Die Trägerteilchen (200 g) wurden wie in Vergleichsbeispiel 1 getränkt (nur daß 141 ml statt 66 ml Eisessig verwendet wurden) und getrocknet, so daß die gleiche Dotierung vorlag. Anschließend wurde der Katalysator wie in Vergleichsbeispiel 1 getestet. Die Ergebnisse sind aus der Tabelle ersichtlich.

**Beispiel 2**

Es wurde zunächst, aus $SiO_2$-$Al_2O_3$-Mikrokugeln (97 Gew.-% $SiO_2$, 3 Gew.-% $Al_2O_3$) mit einer Oberfläche von 170 $m^2$/g sowie einem Füllstoff und einem Gleitmittel ein Träger hergestellt. Der fertige Träger hatte ein Porenvolumen von 0,75 ml/g, das zu 58 % von Poren mit 70 - 100 Angström Durchmesser und zu 12 % von Poren mit 200 - 3000 Angström Durchmesser gebildet wurde. Die Trägerteilchen hatten dieselbe Form und Größe wie in Beispiel 1, jedoch hatten sie jetzt eine Oberfläche von 132 $m^2$/g. Die Trägerteilchen (200 g) wurden wie in Vergleichsbeispiel 1 getränkt (nur daß 131 ml statt 66 ml Eisessig verwendet wurden) und getrocknet, so daß die gleiche Dotierung vorlag. Anschließend wurde der Katalysator wie in Vergleichsbeispiel 1 getestet. Die Ergebnisse sind aus der Tabelle ersichtlich.

**Beispiel 3**

Es wurde zunächst ein Kieselsol mit glasigen $SiO_2$-Mikrokugeln (Oberfläche 200 $m^2$/g) versetzt, getrocknet und gebrannt und dadurch ein Träger hergestellt. Der fertige Träger hatte ein Porenvolumen von 0,64 ml/g, das zu 65 % von Poren mit 70 - 100 Angström Durchmesser und zu 9 % von Poren mit 200 - 3000 Angström Durchmesser gebildet wurde. Die Trägerteilchen waren durch Extrudieren gewonnene Strangabschnitte (Durchmesser und Höhe jeweils 6 mm) mit einer Oberfläche von 170 $m^2$/g.

Die Trägerteilchen (200 g) wurden wie in Vergleichsbeispiel 1 getränkt (nur daß 110 ml statt 66 ml Eisessig verwendet wurden) und getrocknet, so daß die gleiche Dotierung vorlag. Anschließend wurde der Katalysator wie in Vergleichsbeispiel 1 getestet. Die Ergebnisse sind aus der Tabelle ersichtlich.

**Beispiel 4**

Es wurde zunächst aus $SiO_2$-Mikrokugeln mit einer Oberfläche von 300 m²/g sowie einem Füllstoff und einem Gleitmittel ein Träger hergestellt. Der fertige Träger hatte ein Porenvolumen von 0,91 ml/g, das zu 56 % von Poren mit 70 - 100 Angström Durchmesser und zu 10 % von Poren mit 200 - 3000 Angström Durchmesser gebildet wurde. Die Trägerteilchen hatten dieselbe Form und Größe wie in Beispiel 1. Die Oberfläche der Trägerteilchen betrug jedoch jetzt 184 m²/g.

Die Trägerteilchen (200 g) wurden wie in Vergleichsbeispiel 1 getränkt (nur daß 163 ml statt 66 ml Eisessig verwendet wurden) und getrocknet, so daß die gleiche Dotierung vorlag. Anschließend wurde der Katalysator wie in Vergleichsbeispiel 1 getestet. Die Ergebnisse sind aus der Tabelle ersichtlich.

Dabei bedeutet "Beitrag (%)" den prozentualen Beitrag zum Porenvolumen, den die Poren des Durchmessers 70 - 100 Angström bzw. 200 - 3000 Angström liefern.
"RZA" bedeutet die Raum- Zeit-Ausbeute;
"Verbrennung (%)" bedeutet den Prozentsatz des umgesetzten Ethylens, der in $CO_2$ umgewandelt wird, und
"Ethylacetatgehalt" bezieht sich auf den Gehalt des kondensierten Teils des Reaktionsproduktes an Ethylacetat, das als Nebenprodukt entsteht.

| | Vergleichs- beispiel 1 | Vergleichs- beispiel 2 | Beispiel 1 | Beispiel 2 | Beispiel 3 | Beispiel 4 |
|---|---|---|---|---|---|---|
| Beitrag (%) 70 - 100 Å-Poren | 8 | 21 | 62 | 58 | 73 | 56 |
| 200 - 3000 Å-Poren | 29 | 42 | 9 | 12 | 9 | |
| RZA (g/l·h) | 440 | 742 | 773 | 763 | 722 | 769 |
| Verbrennung (%) | 14 | 15 | 6 | 8 | 10 | 6 |
| Ethylacetatgehalt (ppm) | 260 | 280 | 160 | 184 | 170 | 230 |

**Patentansprüche**

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, sowie gegebenenfalls zusätzlich Gold und/oder Goldverbindungen, sowie als Aktivatoren Alkaliverbindungen und gegebenenfalls zusätzlich Cadmiumverbindungen auf einem Träger enthält, der aus $SiO_2$ oder $SiO_2$-$Al_2O_3$-Gemisch mit einer Oberfläche von 50 - 250 $m^2$/g und einem Porenvolumen von 0,4 - 1,2 ml/g besteht und eine Korngröße von 4 bis 9 mm hat, dadurch gekennzeichnet, daß 5 bis 20 % des Porenvolumens des Trägers von Poren mit Radien von 200 bis 3000 Angström und 50 bis 90 % des Porenvolumens von Poren mit Radien von 70 bis 100 Angström gebildet wird, wobei Katalysatoren ausgeschlossen sind, die Palladium und Gold als Metalle enthalten und mehr als 90 Gew.% jedes dieser beiden Metalle im äußeren Teil der Trägerteilchen vorliegen, der bis zu einer Tiefe von 30% der Trägerteilchen reicht.

2. Verfahren nach Anspruch 1, dadurch gekennzeichnet, daß 8 bis 15 % des Porenvolumens von Poren mit Radien von 200 bis 3000 Angström und 55 bis 75 % des Porenvolumens von Poren mit Radien von 70 bis 100 Angström gebildet wird.

3. Katalysator, der Palladium und/oder dessen Verbindungen, sowie gegebenenfalls zusätzlich Gold und/oder Goldverbindungen, sowie als Aktivatoren Alkaliverbindungen und gegebenenfalls zusätzlich Cadmiumverbindungen auf einem Träger enthält, der aus $SiO_2$ oder $SiO_2$-$Al_2O_3$-Gemisch mit einer Oberfläche von 50 - 250 $m^2$/g und einem Porenvolumen von 0,4 - 1,2 ml/g besteht und eine Korngröße von 4 bis 9 mm hat, dadurch gekennzeichnet, daß 5 bis 20 % des Porenvolumens des Trägers von Poren mit Radien von 200 bis 3000 Angström und 50 bis 90 % des Porenvolumens von Poren mit Radien von 70 bis 100 Angström gebildet wird, wobei Katalysatoren ausgeschlossen sind, die Palladium und Gold als Metalle enthalten und mehr als 90 Gew.% jedes dieser beiden Metalle im äußeren Teil der Trägerteilchen vorliegen, der bis zu einer Tiefe von 30% der Trägerteilchen reicht.

4. Katalysator nach Anspruch 3, dadurch gekennzeichnet, daß 8 bis 15 % des Porenvolumens von Poren mit Radien von 200 bis 3000 Angström und 55 bis 75 % des Porenvolumens von Poren mit Radien von 70 bis 100 Angström gebildet wird.

**Claims**

1. A process for the preparation of vinyl acetate in the gas phase from ethylene, acetic acid and oxygen or oxygen containing gases on a catalyst which contains palladium and/or compounds thereof, and additionally, if appropriate, gold and/or gold compounds, and, as activators, alkali metal compounds and additionally, if appropriate, cadmium compounds on a support which comprises $SiO_2$ or an $SiO_2$-$Al_2O_3$ mixture having a surface area of 50-250 $m^2$/g and a pore volume of 0.4-1.2 ml/g, and has a grain size of from 4 to 9 mm, wherein 5 to 20% of the pore volume of the support is formed by pores having radii of from 200 to 3,000 Ångstrom and 50 to 90% of the pore volume is formed by pores having radii of from 70 to 100 Ångstrom, excluding catalysts which comprise palladium and gold as metals and in which more than 90% by weight of each of these two metals is in the outer part of the support particles, extending to a depth of 30% of the support particles.

2. The process as claimed in claim 1, wherein 8 to 15% of the pore volume is formed by pores having radii of from 200 to 3,000 Ångstrom and 55 to 75% of the pore volume is formed by pores having radii of from 70 to 100 Ångstrom.

3. A catalyst which contains palladium and/or compounds thereof, and additionally, if appropriate, gold and/or gold compounds, and, as activators, alkali metal compounds and additionally, if appropriate, cadmium compounds on a support which comprises $SiO_2$ or an $SiO_2$-$Al_2O_3$ mixture having a surface area of 50-250 $m^2$/g and a pore volume of 0.4-1.2 ml/g, and has a grain size of from 4 to 9 mm, wherein 5 to 20% of the pore volume of the support is formed by pores having radii of from 200 to 3,000 Ångstrom and 50 to 90% of the pore volume is formed by pores having radii of from 70 to 100 Ångstrom, excluding catalysts which comprise palladium and gold as metals and in which more than 90% by weight of each of these two metals is in the outer part of the support particles, extending to a depth of 30% of the support particles.

4. A process as claimed in claim 3, wherein 8 to 15% of the pore volume is formed by pores having radii of from 200 to 3,000 Ångstrom and 55 to 75% of the pore volume is formed by pores having radii of from 70 to 100 Ångstrom.

**Revendications**

1. Procédé de fabrication d'acétate de vinyle en phase vapeur, à partir d'éthylène, d'acide acétique et d'oxygène ou de gaz contenant de l'oxygène sur un catalyseur, qui contient du palladium et/ou ses composés, ainsi qu'éventuellement de l'or et/ou des composés de l'or, ainsi que, comme activateurs, des composés alcalins et éventuellement en outre, des composés du cadmium sur un support, qui consiste en $SiO_2$ ou en un mélange de $SiO_2$-$Al_2O_3$ ayant une surface spécifique de 50 à 250 $m^2/g$ et un volume des pores de 0,4 à 1,2 $m^2/g$, et ayant une grosseur des grains de 4 à 9 mm, procédé caractérisé en ce que 5 à 20 % du volume des pores du support sont constitués par des pores ayant des rayons de 200 à 3000 angströms et 50 à 90 % du volume des pores sont constitués par des pores ayant des rayons de 70 à 100 angströms, à l'exclusion de catalyseurs qui contiennent le palladium et l'or sous forme métallique et dont plus de 90 % en poids de chacun de ces deux métaux se trouvent sur la partie extérieure des particules de support, cette partie allant jusqu'à une profondeur de 30 % des particules du support.

2. Procédé selon la revendication 1, caractérisé en ce que 8 à 15 % du volume des pores sont constitués par des pores ayant des rayons de 200 à 3000 angströms et 55 à 75 % du volume des pores sont constitués par des pores ayant des rayons de 70 à 100 angströms.

3. Catalyseur, qui contient, sur un support, du palladium et/ou ses composés, ainsi qu'éventuellement en outre, de l'or et/ou des composés de l'or, ainsi que, comme activateurs, des composés alcalins et éventuellement, en outre, des composés du cadmium, le support consistant en $SiO_2$ ou en un mélange $SiO_2$-$Al_2O_3$ ayant une surface de 50 à 250 $m^2/g$ et ayant un volume des pores de 0,4 à 1,2 $m^2/g$, et une grosseur des grains de 4 à 9 mm, catalyseur caractérisé en ce que 5 à 20 % du volume des pores du support sont formés par des pores ayant des rayons de 200 à 3000 angströms et 50 à 90 % du volume des pores sont constitués par des pores ayant des rayons de 70 à 100 angströms, à l'exclusion de catalyseurs qui contiennent le palladium et l'or sous forme métallique et dont plus de 90 % en poids de chacun de ces deux métaux se situent sur la partie extérieure des particules de support, partie extérieure allant jusqu'à une profondeur de 30 % des particules du support.

4. Catalyseur selon la revendication 3, caractérisé en ce que 8 à 15 % du volume des pores sont formés par des pores ayant des rayons de 200 à 3000 angströms et 55 à 75 % du volume des pores sont formés par des pores ayant des rayons de 70 à 300 angströms.